# EUROPEAN PATENT APPLICATION

(11) **EP 2 584 045 A1**
(43) Date of publication of application: **24.04.2013**
(21) Application number: 11795575.7
(22) Date of filing: 03.06.2011
(51) Int. Cl.: C12Q 1/68

(54) **METHOD FOR DETECTION OF COLORECTAL TUMOR**

(30) Priority: 16.06.2010 JP 2010137460
(71) Applicant: National University Corporation Hamamatsu University School of Medicine, Hamamatsu-shi Shizuoka 431-3192 (JP)
(72) Inventor: KANAOKA Shigeru, Hamamatsu-shi Shizuoka 431-3192 (JP); YOSHIDA Kenichi, Hamamatsu-shi Shizuoka 431-3192 (JP); HAMAYA Yasushi, Hamamatsu-shi Shizuoka 431-3192 (JP)
(74) Representative: Jansen, Cornelis Marinus
(86) International application number: PCT/JP2011/062785
(87) International publication number: WO 2011/158667

(57) **Abstract**

An object of the present invention is to provide a method for detecting a colorectal tumor, and particularly advanced adenoma and early cancer, by using a component contained in stool as an indicator.

Provided is a method for detecting colorectal tumor using a marker gene, comprising: (A) a step for extracting RNA contained in stool collected from a subject, (B) a step for measuring the amount of RNA derived from a marker gene present in the RNA obtained in step (A), and (C) a step for comparing the amount of RNA derived from the marker gene measured in step (B) with preset threshold values for each type of marker gene; wherein, the marker gene is creatine kinase B (CKB) gene.

## Description

### TECHNICAL FIELD

The present invention relates to a method for detecting colorectal tumor using a marker gene, and particularly, to a method for detecting advanced adenoma and early cancer. More specifically, the present invention relates to a method for detecting the presence or absence of colorectal tumor in subjects from whom stool has been collected by using the amount of RNA derived from a marker gene contained in the stool as an indicator.
The present application claims priority from Japanese Patent Application No. 2010-137460, filed in Japan on June 16, 2010, the contents of which are incorporated herein by reference.

### BACKGROUND ART

The number of deaths caused by colorectal cancer is increasing. Colorectal cancer is the fourth leading cause of death among men and the leading cause of death among women among all cancer deaths (2005 Cancer Death Statistics). In addition, according to estimates of the number of persons afflicted with cancer in the year 2020, colorectal cancer is predicted to be the second leading cause of death among men and the leading cause of death among women. Therefore, there is a pressing need for comprehensive measures against colorectal cancer, including secondary prevention. Since colorectal cancer has an extremely high five-year survival rate in comparison with other forms of cancer in the case of early detection and proper treatment, mass screening for colorectal cancer is one of the most effective methods.

In order to make a definitive diagnosis of colorectal cancer, an endoscopic examination is typically performed that enables the large intestine to be viewed directly, after which a biopsy examination of the affected area is additionally performed as necessary. However, since these procedures are invasive and require sophisticated and specialized techniques, they are unsuitable for primary screening in the manner of mass screening.

It is important that a detection method be both simple and noninvasive in order to be used for mass screening. The only noninvasive method able to be used at present is a stool examination for investigating the presence or absence of occult blood, or in other words, an occult blood test, and this method is widely used as a standard method for mass screening for colorectal cancer. However, since the appearance of hemoglobin in stool is not specific to tumors, the occult blood test has the shortcomings of low sensitivity and low specificity (sensitivity: 30% to 90%, specificity: 70% to 98%) as well as significant incidences of false negatives and false positives.

Methods that use components contained in stool as indicators are also used to detect colorectal cancer noninvasively. Since stool may contain cells that have detached from cancer tissue, the composition of stool is considered to be able to reflect gastrointestinal lesions. Therefore, persons with cancer and healthy persons can be distinguished by using genes that are hardly expressed at all in normal tissue but highly expressed in cancer tissue as biomarkers, and using the amount of mRNA of those genes in stool as an indicator of the presence of cancer. In this manner, the use of stool as a specimen makes it possible to eliminate invasiveness and dramatically improve the burden of the examination on the subject.

For example, methods using DNA have been reported that are based on detection of K-ras, p-53 or APC gene mutations present in stool or microsatellite instability and the like (see, for example, Non-Patent Documents 1 to 4). In addition, methods have also been developed that detect mRNA of protein kinase C (PKC) in stool (see, for example, Non-Patent Documents 5 to 7), examine the expression of CD44 variant in the cell fraction of stool (see, for example, Non-Patent Document 8), or detect the presence or absence of methylation of genomic DNA contained in stool (see, for example, Non-Patent Document 9).

In this manner, numerous genes have been reported that can be used as biomarkers capable of detecting colorectal cancer by using the content at which they are present in stool as an indicator. However, sensitivity in the case of using these biomarkers has the problem of only being comparable to or lower than that of the occult blood method. In the case of mass screening in particular, although it is important to detect tumors that can be treated either endoscopically or by surgical resection as in the case of early cancer or advanced adenoma having a high possibility of undergoing malignant transformation, all of the aforementioned biomarkers are inferior to the occult blood method in terms of their detection sensitivity for early cancer and advanced adenocarcinoma. Consequently, there is a strong desire for the development of a method for detecting early cancer with high sensitivity that uses stool for the specimen.

A method has been disclosed by the inventors of the present invention that uses the expression level of cyclooxygenase-2 (COX-2) gene in stool as an indicator as a method for detecting colorectal cancer with higher sensitivity than the occult blood method (see, for example, Patent Documents 1 to 4). Although COX-2 gene is extremely useful as a gene marker for colorectal cancer, there are some colorectal cancers (COX-2-negative colorectal cancer) in which expression levels of COX-2 gene do not increase, and such cases cannot be detected with this method. Although Patent Document 3 discloses gene markers such as matrix metalloproteinease-7 (MMP-7) or Snail gene that can be used in combination with COX-2 gene, since the expression levels of many of these genes demonstrate nearly the same behavior as expression levels of COX-2 gene, even in the case of using these gene markers in combination with COX-2 gene, it is difficult to improve detection sensitivity for COX-2-negative colorectal cancer. In addition, since the expression levels of COX-2 gene, MMP-7 gene and Snail gene in stool tend to increase dependent on the degree of progression of the cancer, they also have the problem of having lower detection sensitivity for early cancer than for advanced cancer.

On the other hand, expression levels and intracellular localization of creatine kinase B (CKB) and heterogeneous nuclear ribonucleoprotein F (hnRNP F) have been reported to change in colorectal cancer (see, for example, Non-Patent Document 10). Expression levels of CKB have also been reported to increase in uterine cancer, and the CKB content of serum has been reported to able to be used as a uterine cancer marker (see, for example, Non-Patent Document 11). However, there are currently no reports describing the potential for the use of the CKB content of stool as a marker for colorectal cancer. A person with ordinary skill in the art would naturally understand that simply encoding a protein for which the expression level thereof changes dependent on malignant transformation does not guarantee that a gene can be used as a clinically useful biomarker.

### [Prior Art Documents]

### [Patent Documents]

[Patent Document 1] Japanese Patent No. 4134047
[Patent Document 2] Japanese Patent No. 4206425
[Patent Document 3] International Publication No. WO 2007/018257
[Patent Document 4] International Publication No. WO 2008/093530

### [Non-Patent Documents]

[Non-Patent Document 1] D. Sidransky, et al., Science, 1992, Vol. 256, pp. 102-105
[Non-Patent Document 2] S.M. Dong, et al., Journal of the National Cancer Institute, 2001, Vol. 93, No. 11, pp. 858-865
[Non-Patent Document 3] G. Traverso, et al., The New England Journal of Medicine, 2002, Vol. 346, No. 5, pp. 311-320
[Non-Patent Document 4] G. Traverso, et al., The Lancet, 2002, Vol. 359, pp. 403-404
[Non-Patent Document 5] L.A. Davidson, et al., Carcinogenesis, 1998, Vol. 19, No. 2, pp. 253-257
[Non-Patent Document 6] R.J. Alexander and R.F. Raicht, Digestive Diseases and Sciences, 1998, Vol. 43, No. 12, pp. 2652-2658
[Non-Patent Document 7] T. Yamao, et al., Gastroenterology, 1998, Vol. 114, No. 6, pp. 1196-1205
[Non-Patent Document 8] H. Saito, Japanese Journal of Cancer Research, 1996, Vol. 87, No. 10, pp. 1011-1024
[Non-Patent Document 9] T. Nagasaka, et al., Journal of the National Cancer Institute, 2009, Vol. 101, No. 18, pp. 1244-1258
[Non-Patent Document 10] M. Balasubramani, et al., Cancer Research, 2006, Vol. 66, No. 2, pp. 763-769
[Non-Patent Document 11] H.G. Huddleston, et al., Gynecologic Oncology, 2005, Vol. 96, pp. 77-83

### DISCLOSURE OF THE INVENTION

### [Problems to be Solved by the Invention]

An object of the present invention is to provide a method for detecting colorectal tumor, and particularly advanced adenoma and early cancer, with high sensitivity by using a component contained in stool as an indicator.

### [Means for Solving the Problems]

As a result of conducting extensive studies to solve the aforementioned problems, the inventors of the present invention found that, when RNA was extracted from stool provided by persons with a colorectal tumor and RNA derived from human genes contained in the RNA was analyzed, the amount of RNA derived from creatine kinase B (CKB) gene contained in the stool was greater in persons having a colorectal tumor than in persons not having a colorectal tumor (persons free of any particular disease in the large intestine), thereby leading to completion of the present invention.

Namely, the present invention is composed in the manner described below.
(1) A method for detecting a colorectal tumor using a marker gene, comprising:
   (A) a step for extracting RNA contained in stool collected from a subject,
   (B) a step for measuring the amount of RNA derived from a marker gene present in the RNA obtained in step (A), and
   (C) a step for comparing the amount of RNA derived from the marker gene measured in step (B) with preset threshold values for each type of marker gene; wherein,
   the marker gene is creatine kinase B (CKB) gene.
(2) The method for detecting a colorectal tumor described in (1) above, wherein one or more types of genes selected from the group consisting of cyclooxygenase-2 (COX-2) gene, matrix metalloproteinase-7 (MMP-7) gene, Snail gene, matrix metalloproteinase-1 (MMP-1) gene and β2 microglobulin (B2M) gene are further used as the marker gene.
(3) The method for detecting a colorectal tumor described in (1) above, wherein COX-2 gene is further used as the marker gene.
(4) The method for detecting a colorectal tumor described in (3) above, wherein one of more types of genes selected from the group consisting of MMP-7 gene, Snail gene, MMP-1 gene and B2M gene is further used as the marker gene.
(5) The method for detecting a colorectal tumor described in (1) above, wherein MMP-7 gene is further used as the marker gene.
(6) The method for detecting a colorectal tumor described in any one of (3) to (5) above, wherein colorectal adenoma or early colorectal cancer is detected.
(7) The method for detecting a colorectal tumor described in any one of (1) to (5) above, wherein the subject has been diagnosed as having a colorectal tumor, and steps (A) to (C) are respectively carried out on stool collected from the subject over time to monitor the possibility of recurrence of a colorectal tumor in the subject.
(8) A gene marker for a colorectal tumor composed of creatine kinase B (CKB) gene.
(9) A kit for detecting a colorectal tumor using stool, comprising:
   a device or reagent for extracting RNA contained in stool, and
   at least either a probe or primer for detecting RNA derived from creatine kinase B (CKB) gene.

### [Effects of the Invention]

Use of the method for detecting a colorectal tumor of the present invention makes it possible to provide information useful for judging whether or not a subject has a colorectal tumor by using stool collected from the subject as a specimen.

### BRIEF DESCRIPTION OF THE DRAWINGS

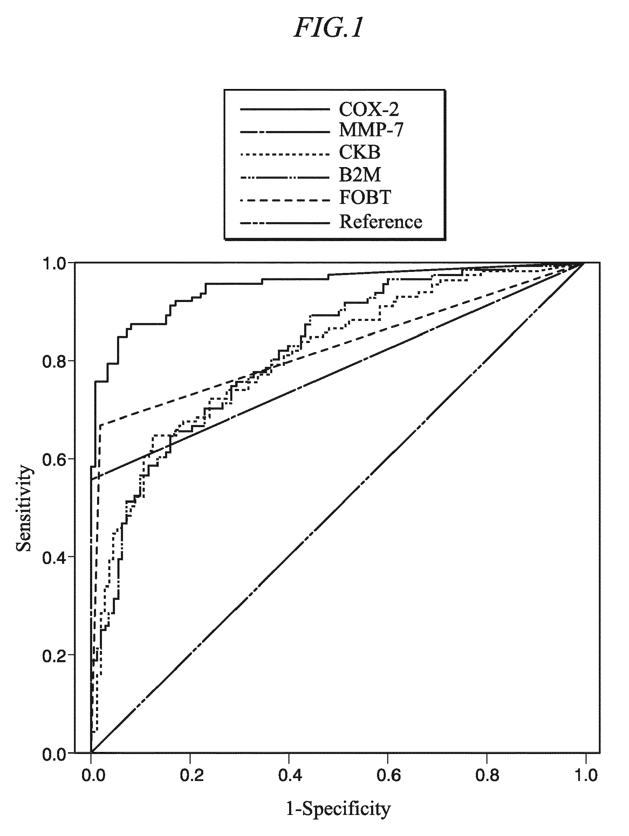

FIG. 1 is a drawing showing the results of analyzing receiver operating characteristic (ROC) in the case of using the amount of RNA derived from each marker gene in stool obtained in Example 1 as a gene marker for colorectal tumor.

### BEST MODE FOR CARRYING OUT THE INVENTION

In the present invention and description of the present application, a colorectal tumor refers to a tumor that forms in the large intestine, regardless of whether it is benign or malignant, and includes both colorectal adenoma and colorectal cancer.

The degree of progression of colorectal cancer is an important factor in terms of determining the treatment method. Colorectal cancer is typically classified into one of clinical stages 0 to IV. In the present invention and description of the present application, each stage respectively indicates the states indicated below.
Stage 0: Cancer has not progressed beyond mucosal membrane.
Stage I: Cancer has not progressed beyond large intestinal wall.
Stage II: Cancer has progressed beyond proper muscle layer of large intestinal wall and is extending outside the wall.
Stage III: Cancer has metastasized to lymph nodes.
Stage IV: Cancer has metastasized to distant organs and lymph nodes.

Early cancer and advanced cancer are defined according to the depth of tumor invasion. Early cancer refers to that which is localized in the mucosal membrane or submucosal layer of the large intestinal wall without going there beyond, and indicates clinical stage 0 and a portion of clinical stage I colorectal cancer. Advanced cancer refers to that in which the invasive front of the cancer has gone beyond the submucosal layer and reached the proper muscle layer or deeper, and indicates a portion of clinical stage I, clinical stage II, clinical stage III and clinical stage IV colorectal cancer. These classifications are defined in the 7th Edition of the Japanese Classification of Colorectal Cancer (Japanese Society for Cancer of the Colon and Rectum, Kanehara & Co., Ltd., 2006).

Colorectal adenoma is divided into small adenoma and advanced adenoma according to size and histological grade. It is difficult to differentiate colorectal adenoma from early colorectal cancer and from mucosal cancer in particular. In particular, advanced adenoma more than 10 mm in size is considered to be equivalent to cancer since it is a tumor having the potential to develop into cancer of the submucosal layer (stage 1 cancer) at some point in the future in the same manner as mucosal cancer. Accordingly, it is important in primary screening such as mass screening to detect colorectal adenoma, and particularly advanced adenoma, in the same manner as early colorectal cancer.

In addition, in the present invention and description of the present application, the phrase "RNA derived from a marker gene" refers to RNA transcribed from the entire length of genomic DNA or a portion thereof of a marker gene, and may be mRNA of that gene or a portion of that mRNA (fragment).

In the present invention and description of the present application, a "person not having cancer" refers to a person who does not have a colorectal tumor, and includes not only healthy persons, but also persons having a disease other than a colorectal tumor.

### <CKB Gene>

As was previously clearly determined by the inventors of the present invention, the amount of RNA derived from COX-2 gene in stool is an extremely effective biomarker for detecting colorectal cancer (see Patent Documents 1 to 4). However, in the case of using only COX-2 gene as a biomarker, colorectal cancer negative for COX-2 cannot be detected. Therefore, the inventors of the present invention believed that colorectal tumors could be detected with good sensitivity in mass screening and the like by combining COX-2 gene with a marker gene enabling the detection of COX-2-negative colorectal cancer, and conducted a search for such a novel marker gene.

More specifically, total RNA was extracted from stool specimens respectively collected from patients having been definitively diagnosed as having colorectal cancer by endoscopic examination and the like and in whom the amount of RNA derived from COX-2 gene in the stool was extremely high in comparison with healthy persons (strongly COX-2-positive colorectal cancer patients), and patients in whom the amount of RNA derived from COX-2 gene was only roughly equal to that of non-colorectal cancer patients, after which expression of each gene was analyzed using that total RNA. A similar analysis was carried out on stool specimens collected from healthy persons. Furthermore, oral or written informed consent was obtained in advance from the patients and healthy persons. In addition, storage of the collected stool specimens and extraction of RNA were carried out in the same manner as the method described in Example 1 to be subsequently described.

Analysis of the expression of each gene was carried out with the Agilent Expression Array (ordered from the Dragon Genomics Center of Takara Bio Inc. ) using the GeneChip® array. As a result, in contrast to the amount of RNA derived from COX-2 gene present in the stool specimens of strongly COX-2-positive colorectal cancer patients being 25.3 times that of healthy persons, it was only 1.4 times that of healthy persons in COX-2-negative colorectal cancer patients. In addition, the amounts of RNA derived from MMP-7 gene and MMP-1 gene were higher in strongly COX-2-positive colorectal cancer patients, while amounts in COX-2-negative colorectal cancer patients were clearly not higher in comparison with healthy persons in the same manner as COX-2 gene. On the other hand, expression levels of CKB gene in stool specimens from COX-2-negative colorectal cancer patients were found to have increased to 28.8 times the expression level in the stool of healthy persons. On the basis of these results, since the amount of RNA derived from CKB gene in stool tends to be higher in persons with a colorectal tumor than in persons not having a colorectal tumor, it was clearly determined that CKB gene enables the detection of not only COX-2-positive colorectal tumors, but also COX-2-negative colorectal tumors, by using as a marker gene for colorectal tumors.

### <Method for Detecting Colorectal Tumor>

The method for detecting colorectal tumor of the present invention is characterized by the use of CKB gene as a gene marker for colorectal tumor. The amount of RNA derived from CKB gene in stool tends to be higher in persons with a colorectal tumor than in persons not having a colorectal tumor. Consequently, the presence or absence of colorectal tumor can be detected by using the amount of RNA derived from CKB gene in stool as an indicator. Namely, the present invention can be said to be a method for detecting RNA derived from a gene marker for colorectal tumor present in stool by using CKB gene.

In the method for detecting colorectal tumor of the present invention, another marker gene may be used in combination with CKB gene as a gene marker for colorectal tumor. The combined use of two or more types of genes makes it possible to detect colorectal tumor with higher accuracy. There are no particular limitations on other marker genes used in combination with CKB gene provided there is a significant difference in the amounts of RNA derived from those genes in stool between a colorectal tumor afflicted group and non-afflicted group.

In the present invention, one or more types of genes selected from the group consisting of COX-2 gene, MMP-7 gene, Snail gene, MMP-1 gene and B2M gene are preferably used as marker genes used in combination with CKB gene.

Since CKB gene is a gene for which the amount of RNA derived from that gene present in stool is higher in COX-2-negative colorectal cancer patients (colorectal cancer patients in whom the amount of RNA derived from COX-2 gene is roughly only equal to that in persons not having colorectal cancer) than in persons not having colorectal cancer, it was found to be able to be used as a gene marker for colorectal tumor. Accordingly, in the method for detecting colorectal tumor of the present invention, the use of CKB gene in combination with COX-2 gene as marker genes for colorectal tumor is particularly preferable. The combined use of CKB gene with COX-2 gene makes it possible to further improve the detection sensitivity of colorectal adenoma and early cancer.

Specific examples of combinations of colorectal tumor marker genes used in the present invention include the combination of CKB gene and COX-2 gene, the combination of CKB gene, COX-2 gene and MMP-7 gene, the combination of CKB gene, COX-2 gene and Snail gene, the combination of CKB gene, COX-2 gene and MMP-1 gene, the combination of CKB gene, COX-2 gene and B2M gene, the combination of CKB gene, COX-2 gene, MMP-7 gene and B2M gene, and the combination of CKB gene and MMP-7 gene.

For example, by using a combination of three genes, the combination of CKB gene, COX-2 gene and MMP-7 gene makes it possible to improve the detection sensitivity of colorectal adenoma in particular in comparison with the case of the combination of CKB gene, COX-2 gene and Snail gene, the combination of CKB gene, COX-2 gene and MMP-1 gene, or the combination of CKB gene, COX-2 gene and B2M gene. In addition, the combined use of the four genes of CKB gene, COX-2 gene, MMP-7 gene and B2M gene in particular makes it possible to detect colorectal tumor with extremely high accuracy and sensitivity.

More specifically, the method for detecting colorectal tumor of the present invention comprises the following steps (A) to (C):
(A) a step for extracting RNA contained in stool collected from a subject,
(B) a step for measuring the amount of RNA derived from a marker gene present in the RNA obtained in step (A), and
(C) a step for comparing the amount of RNA derived from the marker gene measured in step (B) with preset threshold values for each type of marker gene.
The following provides an explanation of each step.

First, in step (A), RNA contained in stool collected from a subject is extracted. In this step, the extracted RNA may be purified by ordinary methods. There are no particular limitations on the methods used to extract and purify RNA from stool, any method known in the relevant technical field may be used, and a commercially available purification kit and the like can also be used. Furthermore, prior to proceeding to the next step, the amount and concentration of RNA obtained in step (A) may be measured in advance. There are no particular limitations on the methods used to measure the amount and concentration of RNA, and any method known in the relative technical field, such as measurement of absorbance, may be used.

There are no particular limitations on the stool supplied for extraction of RNA in step (a) provided it is obtained from a human subject, and for example, a specimen collected for the purpose of a regular health examination or diagnosis and the like can be used. In addition, the stool specimen may be that collected immediately after voiding or that which has been stored for a fixed period of time after collection. There are no particular limitations on the method used to store the stool, and a storage method may be used that is used to store stool specimens for clinical testing and the like. For example, stool that has been frozen or refrigerated may be used for RNA extraction, or stool that has been stored either immersed or suspended in various types of storage solutions may be used. A solution that allows stool to be stored while inhibiting damage to RNA present in the stool, such as a stool sample preparation solution having aqueous alcohol and the like for the active ingredient thereof (see, for example, International Publication No. WO 2010-024251), is preferably used for the storage solution added to stool.

The RNA extracted in step (A) may be used directly in step (B) or may be used in step (B) after having stored for a fixed period of time. The RNA may be stored by any method provided it is a method that enables RNA to be stored while inhibiting decomposition thereof, and for example, may be stored after lyophilization or may be stored in the state of a solution dissolved in purified water.

Next, in step (B), the amount of RNA derived from a marker gene present in the RNA obtained in step (A) is measured. There are no particular limitations on the method used to measure the amount of RNA derived from the marker gene in step (B), and can be suitably selected from among known techniques typically used in the case of measuring the amount of nucleic acid having a specific base sequence.

Furthermore, in the present invention and description of the present application, measurement of the amount of RNA refers not only to strict quantification, but also refers to semi-quantitative measurement or measuring to a degree that allows a quantitative comparison with a prescribed threshold value and the like. For example, the amount of RNA can be calculated based on a calibration curve prepared from the detection results of a control sample having a known concentration based on detection results obtained by detecting RNA derived from a marker gene using a technique known in the relevant technical field. There are no particular limitations on the method used to detect RNA derived from a marker gene, and any method known in the relevant technical field may be used. For example, RNA may be detected by a hybridization method using a probe capable of hybridizing with RNA derived from a marker gene, or RNA may be detected by a method that uses a nucleic acid amplification reaction using polymerase and a primer capable of hybridizing with RNA derived from a marker gene. In addition, a commercially available detection kit and the like can also be used.

In the measurement of step (B), RNA derived from a marker gene present in the RNA obtained in step (A) may be directly detected quantitatively, or RNA derived from a marker gene present in the RNA may be detected quantitatively after having amplified with a nucleic acid amplification reaction. For example, a method can be used that uses two probes that hybridize adjacent to RNA derived from a marker gene, followed by joining with a ligase reaction following hybridization and quantitatively detecting the resulting conjugate, or a method can be used that uses northern blotting using a labeled probe, followed by directly detecting RNA derived from a marker gene by a method that quantitatively detects the amount of probe that has formed a conjugate as a result of hybridization.

Since RNA derived from a marker gene is only present in a trace amount, it can also be measured by a method that uses a nucleic acid amplification reaction. For example, after having synthesized cDNA for the entire length or a portion of the RNA obtained in step (A) by carrying out a reverse transcription reaction, by carrying out a nucleic acid amplification using the resulting cDNA as a template, RNA derived from a marker gene can be detected and the amount thereof can be measured. Although a polymerase chain reaction (PCR) is normally used for nucleic acid amplification using cDNA as a template, examples of other methods that can be used include loop-mediated isothermal amplification (LAMP) and isothermal and chimeric primer-initiated amplification of nucleic acids (ICAN). In addition, by carrying out a quantitative PCR technique such as real-time PCR for the nucleic acid amplification, RNA derived from a marker gene can be easily quantified simultaneous to the detection thereof. In addition, RNA derived from a marker gene can also be amplified by nucleic acid sequence-based amplification (NASBA) in which RNA is amplified directly from RNA. The amplification product of RNA derived from a marker gene can be quantified by a technique commonly known in the relevant technical field. For example, the amplification product can be measured quantitatively by suitably specifically isolating the amplification product by gel or capillary electrophoresis and the like followed by detection thereof.

In addition, various types of sensitization methods such as the Invader® assay can also be used to detect RNA derived from a gene marker. A sensitization method can be used in the case of directly detecting RNA derived from a marker gene present in the RNA obtained in step (A), or in the case of detecting after having amplified with a nucleic acid amplification reaction.

In the case of using a combination of CKB gene with another gene for the marker genes, the amount of RNA derived from each marker gene may be measured separately or simultaneously. For example, an amplification product may be obtained by carrying out PCR separately for each type of gene by using cDNA obtained by a reverse transcription reaction from the entire amount or a portion of the RNA obtained in step (A), or the amplification products of a plurality of genes may be obtained in a single reaction by carrying out multiplex PCR and the like.

In step (C) following step (B), a comparison is made between the amount of RNA derived from a marker gene measured in step (B) and preset threshold values for each type of marker gene. The threshold values are threshold values for differentiating a colorectal cancer or advanced adenoma afflicted group from a non-afflicted group. Cases in which the measured amount of RNA derived from a marker gene is higher than the preset threshold values are taken to be positive, while cases in which that amount is lower than the threshold values are taken to be negative.

The threshold values used in step (C) can be suitably set by a person with ordinary skill in the art in consideration of such factors as the type of method used to measure RNA derived from a marker gene in step (B) or by carrying out a required preliminary examination and the like. For example, the amount of RNA derived from a marker gene can be determined using the same measurement method as step (B) for stool collected from a population known to not have a colorectal disease (non-afflicted group) and stool collected from a population known to have colorectal cancer or advanced colorectal adenoma (afflicted group) based on the results of an endoscopic examination or other examination method, and threshold values for distinguishing between both groups can be suitably set by comparing the measured values of both populations.

When setting threshold values, desired detection accuracy can also be taken into consideration. In cases in which the distribution of the amount of RNA derived from a marker gene in stool has been clearly determined for both a non-afflicted group and an afflicted group, threshold values can be set so that, for example, the probability at which the amount of RNA derived from a marker gene present in stool collected from a person having colorectal cancer or advanced colorectal adenoma is less than the threshold value (namely, the probability of that person being judged to be a non-afflicted person) is within a desired range (such as 10% or less, preferably 5% or less, more preferably 2.5% or less, even more preferably 1% or less, and particularly preferably 0%).

In addition, in the case the distribution of the amount of RNA derived from a marker gene in stool has been clearly determined for only a non-afflicted group, such as in the case of assuming that a subject is a non-afflicted person, threshold values can be set so that the amount of RNA derived from a marker gene in stool collected from that subject is a desired value in terms of a percentile of non-afflicted persons (such as the 90 percentile, preferably the 95 percentile, more preferably the 97.5 percentile, even more preferably the 99 percentile, and particularly preferably the 100 percentile). In addition, threshold values can also be set so that the significance level (p value) at which the amount of RNA derived from a marker gene in stool collected from that subject is less than the threshold value is a desired value (such as 10%, preferably 5%, more preferably 1% and even more preferably 0.1%). Furthermore, the p value may be two-sided or one-sided. Threshold values can also be set in the same manner in the case distribution of the amount of RNA derived from a marker gene in stool has been clearly determined for an afflicted group only. Furthermore, the p value can be determined using a statistical technique such as Mann-Whitney's U test.

More specifically, in the case the amount of RNA derived from a marker gene measured in step (B) (to be referred to as the amount of CKB-derived RNA) is higher than a preset threshold value, the subject is judged to be CKB-positive. In the case of being lower than the threshold value, the subject is judged to be CKB-negative. The amount of CKB-derived RNA tends to be higher in a colorectal tumor-afflicted group than in a non-afflicted group. Consequently, a subject in which a detection result of CKB-positive has been obtained has a high possibility of having a colorectal tumor. Consequently, a definitive diagnosis can be made by using the method for detecting colorectal tumor of the present invention in primary screening such as mass screening, and carrying out an endoscopic examination and the like on a subject judged to be CKB-positive. In this manner, detection results obtained with the method for detecting colorectal tumor of the present invention are useful as information for diagnosing colorectal tumor. In other words, the method for detecting colorectal tumor of the present invention is able to provide information for diagnosing colorectal tumor.

In this manner, the amount of CKB-derived RNA in stool is dependent on the presence or absence of the formation of a colorectal tumor, and tends to be higher in a subject group in which a colorectal tumor has formed than in a subject group in which a colorectal tumor has not formed. Consequently, the method for detecting colorectal tumor of the present invention can also be used to monitor the possibility of recurrence of a colorectal tumor. More specifically, stool specimens are collected over time from a subject who has been diagnosed as having a colorectal tumor. Each of the aforementioned steps (A) to (C) is carried out on each of the collected stool specimens. In the case of, for example, having measured the amount of CKB-derived RNA present in stool over time in a subject in which an affected area of a previously occurring colorectal tumor has been resected by a surgical procedure and the like, in the case the resulting measured value is higher than a preset threshold value, there can be judged to be a high possibility of the colorectal tumor having recurred in the subject at the time the stool specimen was collected.

Sensitivity and specificity in the method for detecting colorectal tumor of the present invention can be suitably adjusted according to the set threshold value. For example, in the case of desiring to obtain adequately high sensitivity, namely in the case of attempting to detect that a subject has a colorectal tumor, the threshold value is preferably set so that the probability of the amount of RNA derived from a marker gene present in stool collected from a person having a colorectal tumor is less than the threshold value (namely, the probability of the subject being judged to be a non-afflicted person) is 1% or less and particularly preferably 0%. On the other hand, in the case of using for primary screening such as during health examinations and the like, specificity is preferably high even if it means sacrificing sensitivity to a certain degree. Consequently, the threshold value can also be set so that the probability of the amount of RNA derived from a marker gene present in stool collected from a healthy person exceeding the threshold value (namely, the probability of the subject being judged to be an afflicted person) is sufficiently small, such as 10% or less and preferably 5% or less. In this manner, in the method for detecting colorectal tumor of the present invention, threshold values can be set according to the desired levels of sensitivity and specificity.

In the case of using a combination of CKB gene and another gene as marker genes, a judgment of positive or negative is made by comparing respective threshold values for each marker gene. A subject positive for at least one marker gene has a high possibility of having a colorectal tumor. Depending on the type of colorectal tumor, even though a certain marker gene among a plurality of colorectal tumor markers is positive, there are many cases in which a different marker gene is negative. Consequently, using a combination of multiple types of marker genes makes it possible to improve the sensitivity of colorectal tumor detection.

In addition, the method for detecting colorectal tumor of the present invention can be carried out more easily by using a kit provided with reagents, devices and the like used in the aforementioned steps (A) and (B). More specifically, such a kit contains devices or reagents for extracting RNA contained in stool and at least a probe or primer for detecting CKB-derived RNA, and is used to detect a colorectal tumor by using stool for the specimen.

Examples of devices or reagents used to extract RNA contained in stool include a suspending solution used to homogenize a collected stool and prepare a suspension in which nucleic acids have been extracted from cells contained in the stool, and a reagent for recovering and purifying RNA from the resulting suspension. The suspending solution can be suitably selected and used from among solutions typically used when recovering nucleic acids from stool. Specific examples of such suspending solutions include phenol solutions and chloroform solutions. In addition, the suspending solution preferably contains an RNase inhibitor such as guanidine thiocyanate, surfactant or chelating agent. Examples of reagents for recovering and purifying RNA from a suspension include ethanol solutions and inorganic supports such as silica.

In addition, since a commercially available nucleic acid purification kit and the like can be used in step (A), a combination of a commercially available nucleic acid purification kit and a probe or primer for detecting CKB-derived RNA can also be used as a kit of the present invention.

An oligonucleotide capable of specifically hybridizing with CKB-derived RNA or a portion of cDNA obtained from that RNA can be used as a probe or primer for detecting CKB-derived RNA. Furthermore, an oligonucleotide capable of hybridizing with CKB-derived RNA and the like may be designed and fabricated using a technique commonly known in the relevant technical field.

For example, in the case of detecting CKB-derived RNA by a method consisting of synthesizing cDNA by a reverse transcription reaction using RNA extracted from stool as a template, followed by carrying out a nucleic acid amplification reaction such as PCR using the resulting cDNA as a template and using a primer for detecting CKB-derived RNA and then detecting the resulting amplification product, the reverse transcriptase, random primer, nucleotide, buffer and the like used in the reverse transcription reaction and the polymerase, labeled nucleotide, non-labeled nucleotide, buffer, PCR device and the like used in PCR may also be contained in the kit of the present invention.
In addition, a stool sampling rod or stool collection container and the like for collecting stool voided from a human or other animal can also be included in the kit of the present invention.

### [Examples]

Although the following provides a more detailed explanation of the present invention by indicating examples thereof, the present invention is not limited to the following examples.

### [Example 1]

### <Stool Sample>

Stool samples were provided by 10 small colorectal adenoma patients (tumor size: 5 mm to 9 mm), 24 advanced colorectal adenoma patients (tumor size: 10 mm or more), 111 colorectal cancer patients (early cancer: 25 patients, advanced cancer: 86 patients), 12 upper gastrointestinal cancer patients (10 gastric tumor patients and 2 esophageal cancer patients) and 113 healthy subjects. Each of the patients had been definitively diagnosed either endoscopically or histologically. In the present example, subjects in which neoplastic lesions (not including adenomatous polyps or hyperplastic polyps measuring less than 5 mm) and clearly inflammatory changes were not observed, and who were free of hemorrhagic lesions, systemic diseases and advanced cancers were used as healthy subjects. In addition, among the 111 colorectal cancer patients, 11 were stage 0, 24 were stage I, 37 were stage II, 25 were stage III and 14 were stage IV. Furthermore, oral or written informed consent was acquired in advance from the patients and healthy subjects.
Specimens (stool samples) were collected 2 to 4 weeks after endoscopic examination or biopsy prior to surgical or endoscopic resection. The collected stool samples were first stored at 4°C, transferred to storage at -80°C within 24 hours after beginning storage, and then stored at that temperature until subjected to RNA extraction.

### <Extraction and Purification of RNA from Stool Samples>

After adding approximately 0.5 g of frozen stool sample and 3 mL of Isogen (Nippon Gene Co., Ltd.) to a sterilized 0.5 mL tube, the contents were mixed and homogenized with a homogenizer. After dispensing approximately 0.7 mL aliquots of the resulting slurry into sterilized 1.5 mL tubes, the tubes were centrifuged for 5 minutes at 12, 000 × g and 4°C, after which the supernatant was dispensed into fresh sterilized 1.5 mL tubes. 0.3 mL of Isogen and 0.3 mL of chloroform were respectively added to each of the tubes, and after vigorously agitating the tubes by vortexing for 30 seconds, the tubes were centrifuged for 15 minutes at 12,000 × g and 4°C. The resulting aqueous phases were carefully recovered from the upper surfaces of the tubes so as not to cause contamination and transferred to fresh 1.5 mL tubes. After adding an equal volume of 70% ethanol solution, the tubes were vigorously agitated by vortexing for 30 seconds. RNA was then extracted and purified from the resulting mixture using the RNeasy Mini Kit (Qiagen K.K.). The purified RNA was quantified using NanoDrop 1000 (NanoDrop Technologies Inc.). The RNA was stored at -80°C until used in subsequent analyses.

### <Measurement of Marker Gene-Derived RNA>

cDNA was synthesized in accordance with the protocol in a reaction having a final volume of 20 µL using the purified RNA, a random hexamer and reverse transcriptase M-MLV (RNase: Takara Bio Inc.).
The amount of cDNA synthesized from RNA derived from various genes present in stool was measured for CKB gene, COX-2 gene, MMP-7 gene, Snail gene, MMP-1 gene and B2M gene present in the cDNA by carrying out quantitative real-time PCR using the synthesized cDNA as template. TaqMan® primer-probe sets commercially available from Applied Biosystems Inc. were respectively used for detecting these marker genes. Furthermore, the probes contained in these sets were reporter probes labeled with the fluorescent material FAM on the 5'-end and labeled with a quenching material on the 3'-end. More specifically, sterilized purified water was added to 1 µL of cDNA solution and 1 µL of 20x TaqMan Primers and Probe Mixture (Applied Biosystems Inc.) to prepare to a final volume of 20 µL, and the resulting solution was used for the PCR reaction solution. PCR solutions respectively prepared for each gene were then subjected to nucleic acid amplification (PCR) while measuring fluorescence intensity on a real-time basis using the Model 7500 Fast Real-Time PCR System (Applied Biosystems Inc.) under reaction conditions consisting of treating for 20 seconds at 95°C followed by treating for 60 cycles consisting of 3 seconds at 95°C and 30 seconds at 62°C. Plasmids containing cDNA of each gene were used as control samples (standard substances) for calculating the number of copies and were amplified simultaneously.
Statistical processing was carried out on the amounts of RNA derived from the marker genes obtained as a result of measurement using Mann-Whitney's U test. In addition, all statistical processing was carried out in the form of two-sided testing and a p value of <0.05 was considered to be statistically significant.
Furthermore, since the majority of marker gene-derived RNA is mRNA derived from that gene, it will hereinafter be referred to as mRNA.

### <Immunochemical Fecal Blood Test (IFOBT (single))>

Immunochemical fetal occult blood tests (MPA) (single) were carried out on the same stool samples used to measure the amounts of marker gene-derived RNA to detect the presence of occult blood. Immunochemical fetal occult blood tests were carried out in accordance with the protocol provided using a commercially available occult blood kit (MagStream® HemSp-N, magnetic particle agglutination reaction reagent, Fujirebio Inc., Serial No. 214794).

### <Results of Measuring Amount of mRNA of each Marker Gene>

The numbers of copies of mRNA of each marker gene are shown in Table 1. In Table 1, the upper numbers indicate average values while the lower numbers indicate the range. In addition, the category of "Other Cancer" indicates the results for patients with upper gastrointestinal cancer. As a result, the amount of CKB mRNA in stool was determined to be significantly higher in the colorectal tumor afflicted groups consisting of patients with colorectal cancer and advanced colorectal adenoma than in the healthy control group. Namely, on the basis of these results, setting suitable threshold values clearly demonstrated that colorectal tumors can be detected by using the amount of CKB-derived RNA in stool as an indicator. In particular, the amount of CKB mRNA can be said to enable detection of advanced colorectal adenoma since the number of copies in the advanced colorectal adenoma patient group was significantly higher than the healthy control group to a greater degree than COX-2 and the like.

**[Table 1]**

| (copy's number) | | | | | | |
|---|---|---|---|---|---|---|
| | CKB | COX-2 | MMP-7 | Snail | MMP-1 | B2M |
| Cancer | 1.3×104 (0~5.3×10⁵) | 2.2×10⁴ (0~6.4×10⁵) | 101 (0~2.3×10³) | 98 (0~1.8×10³) | 3.5×10³ (0~3.9×10⁶) | 6.5×10⁴ (0~1.6×10⁷) |
| Advanced adenoma | 1.8×10³ (0~1.5×10⁴) | 80 (0~811) | 10 (0~62) | 2 (0~26) | 18 (0~115) | 1.5×10⁴ (1.3×10³~4.4×10⁴) |
| Healthy Control | 6.4×10² (0~4.5×10⁴) | 6 (0~158) | 0 (0~0) | 0 (0~6) | 5 (0~101) | 3.2×10³ (0~3.8×10⁴) |
| Other Cancer | 2.7×10² (0~6.9×10²) | 12 (0~73) | 0 (0~0) | 2 (0~7) | 4 (0~18) | 3.7×10³ (6.1×10²~8.1×10³) |
| Small adenoma | 2.9×10² (0~1.3×10³) | 7 (0~34) | 0 (0~0) | 1 (0~4) | 1 (0~11) | 1.9×10³ (0~1,1×10⁴) |

### <Setting of Cutoff Values>

The numbers of copies of each marker gene in a healthy control group, colorectal cancer group and advanced colorectal adenoma group were analyzed in order to set threshold values (cutoff values) for distinguishing between persons afflicted with a colorectal tumor and non-afflicted persons for each marker gene.
Table 2 shows the average value, standard deviation (SD), median value, 95 percentile value and 97.5 percentile value of the number of copies of each marker gene in the healthy control group. In addition, Tables 3 and 4 show the average value, standard deviation (SD), median value and 25 percentile value of the number of copies of each gene marker in the colorectal cancer group and advanced colorectal adenoma group. On the basis of these results, the cutoff value for CKB gene was set at 1450, that for COX-2 gene was set at 58, that for MMP-7 gene was set at 5, that for Snail gene was set at 9, that for MMP-1 gene was set at 37 and that for B2M gene was set at 21000.

**[Table 2]**

| Healthy Control (copy's number) | | | | | | |
|---|---|---|---|---|---|---|
| | CKB | COX-2 | MMP-7 | Snail | MMP-1 | B2M |
| Average | 636.0 | 6.4 | 0.0 | 0.2 | 4.6 | 3178.4 |
| SD | 4267.5 | 17.3 | 0.0 | 0.9 | 14.5 | 5406.3 |
| Median | 116.1 | 1.0 | 0.0 | 0.0 | 0.0 | 1465.1 |
| 95%ile | 819.8 | 23.9 | 0.0 | 0.9 | 22.4 | 13472.4 |
| 97.5%ile | 1220.3 | 43.6 | 0.0 | 3.8 | 31.3 | 20533.1 |

**[Table 3]**

| Cancer (copy's number) | | | | | | |
|---|---|---|---|---|---|---|
| | CKB | COX-2 | MMP-7 | Snail | MMP-1 | B2M |
| Average | 13107.7 | 21679.0 | 101.3 | 98.3 | 35424.9 | 64675.0 |
| SD | 65218.4 | 93565.2 | 332.6 | 294.1 | 369509.6 | 216211.7 |
| Median | 661.1 | 253.6 | 8.0 | 4.4 | 27.7 | 8813.3 |
| 25%ile | 219.1 | 62.9 | 0.0 | 0.0 | 0.0 | 2824.5 |

**[Table 4]**

| Advanced adenoma (copy's number) | | | | | | |
|---|---|---|---|---|---|---|
| | CKB | COX-2 | MMP-7 | Snail | MMP-1 | B2M |
| Average | 1800.1 | 80.2 | 9.9 | 2.3 | 17.5 | 15305.5 |
| SD | 3337.4 | 168.2 | 18.8 | 5.5 | 25.3 | 13245.5 |
| Median | 705.0 | 23.6 | 0.0 | 0.0 | 8.4 | 10384.6 |
| 25%ile | 353.9 | 7.6 | 0.0 | 0.0 | 0.0 | 5027.1 |

### <Sensitivity and Specificity in Detecting Colorectal Cancer of each Marker Gene>

Each sample was judged to be positive or negative using the cutoff values set as described above, sensitivity and specificity were calculated for detection of colorectatl cancer, and the results were compared with results obtained with the immunochemical fetal occult blood test (IFOBT (single)). The calculation results are shown in Table 5. As a result, although COX-2 gene was better than the immunochemical fetal occult blood test for both sensitivity and specificity, sensitivity of the CKB gene was lower than that of the immunochemical fetal occult blood test although specificity was comparable thereto. In Table 5, "95% CI" indicates the 95% confidence interval (%). Furthermore, calculation of p values for sensitivity and specificity of all subsequent samples was carried out with the χ² test (chi-square test). In addition, all statistical processing was carried out in the form of two-sided testing, and a p value of <0.05 was considered to be statistically significant.

**[Table 5]**

| | CKB | COX-2 | MMP-7 | Snail | MMP-1 | B2M | IFOBT (single) |
|---|---|---|---|---|---|---|---|
| Sensitivity | 28.8% (32/111) | 75.7% (84/111) | 55.9% (62/111) | 42.3% (47/111) | 45.9% (51/111) | 24.3% (27/111) | 66.7% (74/111) |
| 95% Cl | 20.6∼38.2% | 66.6∼83.3% | 46.1∼65.3% | 33.0∼52.1% | 36.4∼55.7% | 16.7∼33.4% | 57.1∼75.3% |
| | | P=0.18 | | | | | |
| | | | | | | | |
| Specificity | 98.2% (111/113) | 99.1% (112/113) | 100% (113/113) | 100% (113/113) | 98.2% (111/113) | 98.2% (111/113) | 98.2% (111/113) |
| 95% Cl | 93.8∼99.8% | 95.2∼100% | 97.4∼100% | 97.4∼100% | 93.8∼99.8% | 93.8∼99.8% | 93.8∼99.8% |
| | P = 1 | P = 1 | P = 0.48 | P = 0.48 | P = 1 | P = 1 | |

### <ROC Analysis of each Marker Gene>

Analyses of receiver operating characteristic (ROC) were carried out in order to investigate the performance of each marker gene as a marker for detection of colorectal tumor. ROC analysis curves were generated using PASW Statistics Ver. 18 (IBM Corp.). The analysis results are shown in Table 6 and FIG. 1. In FIG. 1, ROC curves were generated by plotting sensitivity on the vertical axis and (1-specificity) on the horizontal axis.

**[Table 6]**

| **Area Under Curve** | | | | | |
|---|---|---|---|---|---|
| **Test Result Variable** | **Area** | **Standard Error^{a}** | **Convergent Significant Probability^{b}** | **Convergent 95% Confidence Interval** | |
| | | | | **Lower Limit** | **Upper Limit** |
| **COX-2** | **0.949** | **0.015** | **0.000** | **0.921** | **0.978** |
| **MMP-7** | **0.779** | **0.032** | **0.000** | **0.716** | **0.842** |
| **CKB** | **0.810** | **0.029** | **0.000** | **0.754** | **0.886** |
| **B2M** | **0.818** | **0.028** | **0.000** | **0.764** | **0.872** |
| **FOBT** | **0.824** | **0.030** | **0.000** | **0.767** | **0.882** |

| | | | | | |
|---|---|---|---|---|---|
| a: Based on non-parametric hypothesis b: Null hypothesis: true area = 0.5 | | | | | |

As a result, the area under the curve for the amount of CKB-derived RNA was 0.5 or more in the same manner as the amounts of COX-2-derived RNA, MMP-7-derived RNA and B2M-derived RNA, and the immunochemical fetal occult blood test, and was found to be favorable for use as a marker for detecting colorectal tumor.

### <Sensitivity and Specificity in Detecting Colorectal Cancer when Combining Multiple Marker Genes>

Sensitivity and specificity for detecting colorectal cancer in the case of combining CKB gene with other marker genes were calculated and compared. The calculation results are shown in Tables 7 and 8.

**[Table 7]**

| | CKB/COX-2 | CKB/MMP-1 | CKB/MMP-7 | CKB/Snail | CKB/B2M | COX-2/MMP-1 |
|---|---|---|---|---|---|---|
| Sensitivity | 80.2% (89/111) | 54.1% (60/111) | 64.0% (71/111) | 49.5% (55/111) | 33.3% (37/111) | 77.5% (86/111) |
| 95% Cl | 71.5∼87.1% | 44.3∼63.6% | 54.3∼72.9% | 39.9∼59.2% | 24.7∼42.9% | 68.6∼84.9% |
| | P = 0.033 | P = 0.074 | P = 0.78 | P = 0.014 | P < 0.0001 | P = 0.10 |
| | | | | | | |
| Specificity | 97.3% (110/113) | 96.5% (109/113) | 98.2% (111/113) | 98.2% (111/113) | 98.2% (111/113) | 97.3% (110/113) |
| 95% Cl | 92.4∼99.4% | 91.2∼99.0% | 93.8∼99.8% | 93.8∼99.8% | 93.8∼99.8% | 92.4∼99.4% |
| | P = 1 | P = 0.68 | P = 1 | P = 1 | P = 1 | P = 1 |

**[Table 8]**

| | CKB/ COX-2/ MMP-7 | CKB/ COX-2/ Snail | CKB/ COX-2/ MMP-1 | CKB/ COX-2/ B2M |
|---|---|---|---|---|
| Sensitivity | 83.8% (93/111) | 80.2% (89/111) | 81.1% (90/111) | 81.1% (90/111) |
| 95% Cl | 75.6∼90.1% | 71.5∼87.1% | 72.5∼87.9% | 72.5∼87.9% |
| | P = 0.0051 | P = 0.033 | P = 0.022 | P = 0.022 |
| | | | | |
| Specificity | 97.3% (110/113) | 97.3% (110/113) | 95.6% (108/113) | 97.3% (110/113) |
| 95% Cl | 92.4∼99.4% | 92.4∼99.4% | 90.0∼98.5% | 92.4∼99.4% |
| | P = 1 | P = 1 | P = 0.44 | P = 1 |

As a result, in the case of using two types of marker genes in combination, the combination of CKB gene and COX-2 gene demonstrated the greatest sensitivity. In particular, despite CKB gene demonstrating lower detection sensitivity than MMP-1 gene when used alone, the combination of CKB gene and COX-2 gene demonstrated better sensitivity than the combination of MMP-1 gene and COX-2 gene.

### <Sensitivity in Detecting Colorectal Tumor of each Marker Gene in each Stage>

Each sample was judged to be positive or negative using the cutoff values set as described above, sensitivity and specificity were calculated for detection of each stage of colorectal tumor, and the results were compared with results obtained with the immunochemical fetal occult blood test (IFOBT (single)). The calculation results are shown in Table 9. In Table 9, the terms "0_Ca" to "IV_Ca" respectively indicate stages 0 to IV of colorectal cancer. As a result, CKB gene was determined to enable detection of 0 stage colorectal cancer with higher sensitivity than the immunochemical fecal occult blood test.

**[Table 9]**

| Stage | CKB | COX-2 | MMP-7 | Snail | MMP-1 | B2M | IFOBT (single) |
|---|---|---|---|---|---|---|---|
| Advanced adenoma | 25.0 % (6/24) | 33.3 % (8/24) | 33.3 % (8/24) | 4.2 % (1/24) | 12.5 % (3/24) | 25.0 % (6/24) | 29.2 % (7/24) |
| 0_Ca | 18.2% (2/11) | 18.2 % (2/11) | 27.3 % (3/11) | 9.1 % (1/11) | 0% (0/11) | 0% (0/11) | 0 % (0/11) |
| I_Ca | 16.7 % (4/24) | 62.5 % (15/24) | 45.8 % (11/24) | 12.5 % (3/24) | 29.2 % (7/24) | 12.5 % (3/24) | 45.8 % (11/24) |
| II_Ca | 40.5 % (15/37) | 89.2 % (33/37) | 64.9 % (24/37) | 54.1 % (20/37) | 64.9 % (24/37) | 32.4 % (12/37) | 83.8 % (31/37) |
| III_Ca | 32.0 % (8/25) | 88.0 % (22/25) | 60.0 % (15/25) | 52.0 % (13/25) | 44.0 % (11/25) | 24.0 % (6/25) | 88.0 % (22/25) |
| IV_Ca | 21.4% (3/14) | 85.7 % (12/14) | 64.3 % (9/14) | 71.4 % (10/14) | 64.3 % (9/14) | 42.9 % (6/14) | 71.4% (10/14) |

### <Sensitivity in Detecting Colorectal Tumor for Each Stage when Combining Multiple Marker Genes>

Sensitivities in detecting colorectal cancer of each stage in the case of combining CKB gene with other marker genes were calculated and compared. The calculation results are shown in Table 10. As a result, combining CKB gene with other genes, and particularly COX-2 gene and MMP-7 gene, clearly enhanced the detection sensitivity of colorectal tumor even though sensitivity was lower in the case of CKB gene alone. In particular, as a result of further combining MMP-7 gene, Snail gene, MMP-1 gene or B2M gene with CKB gene and COX-2 gene, advanced colorectal adenoma and stage 0 and stage I cancer were determined to be able to be detected with extremely high sensitivity. As a result of setting suitable cutoff values in particular, advanced colorectal adenoma was determined to be able to be detected an extremely high sensitivity of 50% or higher.

**[Table 10]**

| Stage | CKB/ COX-2 | CKB/ MMP-7 | CKB/ COX-2/ MMP-7 | CKB/ COX-2/ Snail | CKB/ COX-2/ MMP-1 | CKB/ COX-2/ B2M |
|---|---|---|---|---|---|---|
| Advanced adenoma | 50.0 % (12/24) | 54.2 % (13/24) | 66.7 % (16/24) | 50.0 % (12/24) | 50.0 % (12/24) | 54.2 % (13/24) |
| | | | P=0.021 | P=0.24 | P=0.24 | P=0.14 |
| 0_Ca | 36.4 % (4/11) | 36.4 % (4/11) | 45.5 % (5/11) | 36.4 % (4/11) | 36.4 % (4/11) | 36.4 % (4/11) |
| | | | P=0.041 | P=0.097 | P=0.097 | P=0.097 |
| I_Ca | 70.8 % (17/24) | 54.2 % (13/24) | 75.0 % (18/24) | 70.8 % (17/24) | 75.0 % (18/24) | 75.0 % (18/24) |
| | | | P=0.077 | P=0.14 | P=0.077 | P=0.077 |
| II_Ca | 89.2 % (33/37) | 73.0 % (27/37) | 91.9 % (34/37) | 89.2 % (33/37) | 89.2 % (33/37) | 89.2 % (33/37) |
| | | | P=0.48 | P=0.73 | P=0.073 | P=0.73 |
| III_Ca | 92.0 % (23/25) | 68.0 % (17/25) | 92.0 % (23/25) | 92.0 % (23/25) | 92.0 % (23/25) | 92.0 % (23/25) |
| | | | P=1 | P=1 | P=1 | P=1 |
| IV_Ca | 85.7 % (12/14) | 71.4% (10/14) | 92.9 % (13/14) | 85.7 % (12/14) | 85.7 % (12/14) | 85.7 % (12/14) |
| | | | P=0.32 | P=0.65 | P=0.65 | P=0.645 |

### <Sensitivity in Detecting Cumulative Stages of Colorectal Tumor when Combining Multiple Marker Genes>

Sensitivities in detecting cumulative stages of colorectal tumor in the case of combining CKB gene with other marker genes were calculated and compared with results for the immunochemical fecal occult blood test (IFOBT (single)). The calculation results are shown in Table 11. In Table 11, the terms "Ad∼0_Ca" to "Ad∼IV_Ca" respectively indicate the cumulative stages from advanced colorectal adenoma to each stage of colorectal cancer. In Table 11, the case of using COX-2 gene alone and the case of using the combination of COX-2 gene and MMP-1 gene are shown as comparative controls. On the basis of these results as well, the use of CKB gene in combination with other marker genes was determined to enable detection of colorectal tumor with high sensitivity, while further combining with COX-2 gene, and particularly the case of combining with COX-2 gene and MMP-7 gene, was determined to enable detection of colorectal tumor at extremely high sensitivity.

**[Table 11]**

| Stage | COX-2 | COX-2/ MMP-1 | CKB/ COX-2 | CKB/ MMP-7 | CKB/ COX-2/ MMP-7 | CKB/ COX-2/ MMP-1 | CKB/ COX-2/ B2M | IFOBT (single) |
|---|---|---|---|---|---|---|---|---|
| Advanced adenoma | 33.3 % (8/24) | 33.3 % (8/24) | 50.0 % (12/24) | 54.2 % (13/24) | 66.7 % (16/24) | 50.0 % (12/24) | 54.2 % (13/24) | 29.2 % (7/24) |
| | P=1 | P=1 | P=0.24 | P=0.14 | P=0.021 | P=0.24 | P=0.14 | |
| Ad∼ 0_Ca | 28.6 % (10/35) | 28.6 % (10/35) | 45.7 % (16/35) | 48.6 % (17/35) | 60.0 % (21/35) | 45.7 % (16/35) | 48.6 % (17/35) | 20.0 % (7/35) |
| | P=0.58 | P=0.58 | P=0.042 | P=0.023 | P=0.0015 | P=0.042 | P=0.023 | |
| Ad∼ I_Ca | 42.4 % (25/59) | 45.8 % (27/59) | 55.9 % (33/59) | 50.8 % (30/59) | 66.1 % (39/59) | 57.6 % (34/59) | 59.3 % (35/59) | 30.5 % (18/59) |
| | P=0.25 | P=0.13 | P=0.0093 | P=0.039 | P=0.00023 | P=0.0054 | P=0.0031 | |
| Ad∼ II_Ca | 60.4 % (58/96) | 62.5 % (60/96) | 68.8 % (66/96) | 59.4 % (57/96) | 76.0 % (73/96) | 69.8 % (67/96) | 70.8% (68/96) | 51.0 % (49/96) |
| | P=0.25 | P=0.15 | P=0.018 | P=0.31 | P=0.00056 | P=0.012 | P=0.0078 | |
| Ad∼ III_Ca | 66.1 % (80/121) | 67.8 % (82/121) | 73.6 % (89/121) | 61.2 % (74/121) | 79.3 % (96/121) | 74.4 % (90/121) | 75.2 % (91/121) | 58.7% (71/121) |
| | P=0.29 | P=0.18 | P=0.021 | P=0.79 | P=0.00085 | P=0.014 | P=0.0094 | |
| Ad∼ IV_Ca | 68.1 % (92/135) | 69.6 % (94/135) | 74.8 % (101/135) | 62.2 % (84/135) | 80.7 % (109/135) | 75.6 % (102/135) | 76.3 % (103/135) | 60.0 % (81/135) |
| | P=0.20 | P=0.13 | P=0.014 | P=0. 803 | P=0.00032 | P=0.0092 | P=0.0061 | |

Moreover, sensitivity and specificity in detecting colorectal cancer in the case of combining the four genes of CKB gene, COX-2 gene, MMP-7 gene and B2M gene, as well as sensitivity in detecting cumultative stages of colorectal tumor were calculated, and the case of using COX-2 gene only, the case of using the combination of COX-2 gene and MMP-7 gene, the case of using the combination of COX-2 gene and B2M gene, and the case of using the combination of COX-2 gene and CKB gene were compared with results obtained with the immunochemical fetal occult blood test (IFOBT (single)). The calculation results are shown in Tables 12 and 13. As a result, the case of using the combination of CKB gene, COX-2 gene, MMP-7 gene and B2M gene resulting in the highest sensitivity. In particular, sensitivity ini the case of using the combination of these four genes demonstrated an extremely low significance level (p value) of 0.001 or less, thereby suggesting that this combination is sufficiently useful even for clinical testing requiring a high level of accuracy.

**[Table 12]**

| **Comparison of Fecal RNA Test with IFOBT for CRC** | | | | | | |
|---|---|---|---|---|---|---|
| | COX-2 | COX-2/MMP-7 | COX-2/B2M | COX-2/CKB | COX-2/MMP-7/CKB/B2M | IFOBT (single) |
| Sensitivity | 75.7% (84/111) | 80.2% (89/111) | 77.5% (86/111) | 80.2% (89/111) | 84.7% (94/111) | 66.7% (74/111) |
| 95% Cl | 66.7-83.3% | 71.5-87.1% | 68.6-84.9% | 71.5-87.1% | 76.6-90.8% | 57.1-75.3% |
| | P=0.18 | P = 0.033 | P=0.10 | P = 0.033 | P=0.0030 | |
| | | | | | | |
| Specificity | 99.1% (112/113) | 99.1% (112/113) | 97.3% (110/113) | 97.3% (1101113) | 97.3% (110/113) | 98.2% (111/113) |
| 95% Cl | 95.2-100% | 95.2-100% | 92.4-99.4% | 92.4-99.4% | 92.4-99.4% | 93.8-99.8% |
| | P=1 | P=1 | P=1 | P=1 | P=1 | |

**[Table 13]**

| **Sensitivity of Fecal RNA Test and IFOBT according to cummulative Stage** | | | | | | |
|---|---|---|---|---|---|---|
| | COX-2 | COX-2/MMP-7 | COX-2/B2M | COX-2/CKB | COX-2/MMP-7/CKB/B2M | IFOBT (single) |
| Adenoma | 33.3% (8/24) | 50.0% (12/24) | 45.8% (11/24) | 50.0% (12/24) | 70.8 % (17/24) | 29.2% (7/24) |
| | P = 1 | P = 0.24 | P = 0.37 | P = 0.24 | *P = 0.0094* | |
| Ad ∼ 0 ca | 28.6% (10/35) | 45.7% (16/35) | 37.1% (13/35) | 45.7% (16/35) | 62.9% (22/35) | 20.0% (7/35) |
| | P = 0.58 | *P* = *0.042* | P = 0.19 | P = *0.042* | P = *0.00068* | |
| Ad ∼ I ca | 42.4% (25/59) | 54.2% (32/59) | 50.8% (30/59) | 55.9% (33/59) | 69.5% (41/59) | 30.5% (18/59) |
| | P = 0.25 | *P* = *0.015* | *P* = *0.039* | *P* = *0.0093* | *P* = 0. 000051 | |
| Ad ∼ II ca | 60.4% (58/96) | 68.8% (66/96) | 65.6% (63/96) | 68.8% (66/96) | 78.1% (75/96) | 51.0% (49/96) |
| | P = 0.25 | *P = 0.018* | *P* = *0.030* | *P* = *0.018* | *P* = *0.00016* | |
| Ad - III ca | 66.1% (80/121) | 72.7% (88/121) | 70.2% (85/121) | 73.6% (89/121) | 81.0% (98/121) | 58.7% (71/121) |
| | P = 0.29 | *P = 0.030* | P = 0.081 | *P = 0.021* | *P* = *0. 00027* | |
| Ad ∼ IV ca | 68.1% (92/135) | 74.8% (101/135) | 71.9% (97/135) | 74.8% (101/135) | 82.2% (111/135) | 60.0% (81/135) |
| | P = 0.20 | *P = 0.014* | P = 0.054 | *P = 0.014* | *P= 0.000097* | |

### INDUSTRIAL APPLICABILITY

Since the use of the method for detecting colorectal tumor of the present invention makes it possible to accurately test for the presence or absence of colorectal tumor, and particularly advanced colorectal adenoma as well as stage 0 and stage 1 cancer, the method for detecting colorectal tumor of the present invention can be used in fields such as clinical testing using stool samples, and particularly in fields such as clinical testing requiring high levels of reliability and safety.

## Claims

1. A method for detecting a colorectal tumor using a marker gene, comprising:
(A) a step for extracting RNA contained in stool collected from a subject,
(B) a step for measuring the amount of RNA derived from the marker gene present in the RNA obtained in step (A), and
(C) a step for comparing the amount of RNA derived from the marker gene measured in step (B) with preset threshold values for each type of marker gene; wherein,
the marker gene is creatine kinase B (CKB) gene.

2. The method for detecting a colorectal tumor according to claim 1, wherein one or more types of genes selected from the group consisting of cyclooxygenase-2 (COX-2) gene, matrix metalloproteinase-1 (MMP-7) gene, Snail gene, matrix metalloproteinase-1 (MMP-1) gene and β2 microglobulin (B2M) gene are further used as the marker genes.

3. The method for detecting a colorectal tumor according to claim 1, wherein COX-2 gene is further used as the marker gene.

4. The method for detecting a colorectal tumor according to claim 3, wherein one or more types of genes selected from the group consisting of MMP-7 gene, Snail gene, MMP-1 gene and B2M gene are further used as the marker gene.

5. The method for detecting a colorectal tumor according to claim 1, wherein MMP-7 gene is further used as the marker gene.

6. The method for detecting a colorectal tumor according to any one of claims 3 to 5, wherein colorectal adenoma or early colorectal cancer is detected.

7. The method for detecting a colorectal tumor according to any one of claims 1 to 5, wherein the subject has been diagnosed as having a colorectal tumor, and
steps (A) to (C) are respectively carried out on stool collected from the subject over time to monitor the possibility of recurrence of a colorectal tumor in the subject.

8. A gene marker for a colorectal tumor composed of creatine kinase B (CKB) gene.

9. A kit for detecting a colorectal tumor using stool, comprising:
a utensil or reagent for extracting RNA contained in stool, and
at least either a probe or primer for detecting RNA derived from creatine kinase B (CKB) gene.
